# EUROPÄISCHE PATENTSCHRIFT

(11) **EP 0 706 986 B1**
(45) Veröffentlichungstag und Bekanntmachung des Hinweises auf die Patenterteilung: **12.05.1999**
(21) Anmeldenummer: 95115769.2
(22) Anmeldetag: 06.10.1995
(51) Int. Cl.: C07C 57/04, C07C 51/48

(54) **Verfahren zur Abtrennung von (Meth)acrylsäure aus dem Reaktionsgasgemisch der katalytischen Gasphasenoxidation von C3-/C4-Verbindungen**
Process for separating (meth)acrylic acid from the reaction gas mixture of the catalytic oxidation of C3-/C4-compounds in gas phase
Procédé de séparation d'acide (méth)acrylique à partir d'un mélange gazeux réactionnel obtenu par l'oxidation catalytique de composés en C3-/C4

(30) Priorität: 11.10.1994 DE 4436243
(43) Veröffentlichungstag der Anmeldung: 17.04.1996
(73) Patentinhaber: BASF AKTIENGESELLSCHAFT, 67056 Ludwigshafen (DE)
(72) Erfinder: Egly, Horst, D-67459 Böhl-Iggelheim (DE); Diehl, Volker, Dr., D-67158 Ellerstadt (DE); Jörg, Klaus, Dr., D-67117 Limburgerhof (DE)

(56) Entgegenhaltungen:
- DE-A- 2 241 714
- DE-A- 2 449 780
- DE-A- 2 544 930

## Beschreibung

Die vorliegende Erfindung betrifft insbesondere ein neues Verfahren zur Abtrennung von Acrylsäure aus dem Reaktionsgasgemisch der katalytischen Gasphasenoxidation von Propylen und/oder Acrolein durch Gegenstromabsorption mit einer hochsiedenden inerten hydrophoben organischen Flüssigkeit, bei dem man das Reaktionsgasgemisch in einer Absorptionskolonne zu der absteigenden hochsiedenden inerten hydrophoben organischen Flüssigkeit im Gegenstrom führt und anschließend aus dem die Acrylsäure enthaltenden Flüssigkeitsablauf der Absorptionskolonne die Acrylsäure rektifikativ abtrennt.

Acrylsäure bildet aufgrund ihrer sehr reaktionsfähigen monoethylenisch ungesättigten Bindung sowie der Säurefunktion ein wertvolles Monomeres zur Herstellung von Polymerisaten, z.B. für als Klebstoffe geeignete wäßrige Polymerisatdispersionen. Desgleichen gilt für Methacrylsäure.

Unter anderem ist Acrylsäure zugänglich durch Gasphasenoxidation von Propylen und/oder Acrolein mit Sauerstoff oder Sauerstoff enthaltenden Gasen in Gegenwart von Katalysatoren (z.B. Multimetalloxiden, die die Elemente Molybdän und Vanadin in oxidischer Form enthalten) bei erhöhter Temperatur sowie infolge der hohen Reaktionswärme vorzugsweise unter Verdünnen der Reaktionspartner mit Inertgasen wie N₂, CO₂ und/oder Kohlenwasserstoffen und/oder Wasserdampf. Bei diesem Verfahren wird jedoch nicht reine Acrylsäure, sondern ein Reaktionsgasgemisch, das neben Acrylsäure als Nebenkomponenten im wesentlichen nicht umgesetztes Acrolein und/oder Propylen, Wasserdampf (entsteht auch als Reaktionsprodukt), Kohlenoxide, inertes Verdünnungsgas (z.B. Stickstoff, niedere Aldehyde wie Formaldehyd, Maleinsäureanhydrid und insbesondere Essigsäure (vgl. z.B. EP-A 253 409 u. DE-A 19 62 431) enthält, erhalten, von welchem die Acrylsäure anschließend abgetrennt werden muß. Die Gewinnung von Methacrylsäure ist ausgehend von C₄-Verbindungen analog möglich und mit entsprechenden Problemen befrachtet.

Aus der DE-PS 21 36 396 und der DE-A 43 08 087 ist bekannt, Acrylsäure aus dem Reaktionsgasgemisch der katalytischen Gasphasenoxidation von Propylen und/oder Acrolein durch Gegenstromabsorption mit einer hochsiedenden inerten hydrophoben organischen Flüssigkeit abzutrennen.

Das Verfahren wird im wesentlichen so durchgeführt, daß man das Reaktionsgemisch in einer konventionellen Absorptionskolonne zu der absteigenden Absorptionsflüssigkeit im Gegenstrom führt, danach in einer Desorptionskolonne aus dem im wesentlichen aus Acrylsäure, dem Absorptionsmittel und Nebenkomponenten zusammengesetzten Flüssigkeitsablauf der Absorptionskolonne durch Strippen die leichtflüchtigen Nebenkomponenten weitgehend entfernt und anschließend den Flüssigkeitsablauf der Desorptionskolonne zur Abtrennung von im wesentlichen reiner Acrylsäure sowie schwerer flüchtiger Nebenkomponenten rektifikativ behandelt.

Nachteilig an dieser Verfahrensweise ist, daß das Desorberabgas (das gebrauchte Stripgas) im üblichen Betrieb der Desorptionskolonne wenigstens ein Drittel der im der Desorptionskolonne zugeführten Flüssigkeitsablauf der Absorptionskolonne enthaltenen Acrylsäure austrägt, weshalb das Desorberabgas in die Absorberkolonne zurückgeführt werden muß (vgl. Abschnitt 1 auf S. 7 der DE-A 21 36 396). Auf diese Weise führt das Verfahren des Standes der Technik eine nicht unerhebliche Menge der abzutrennenden Acrylsäure ohne Trennwirkung im Kreis, was die Wirtschaftlichkeit der Verfahrensweise des Standes der Technik in erheblichem Ausmaß beeinträchtigt. Das in der DE-A-2241714 offenbarte Gegenstromabsorptionsverfahren vermag infolge der dort geforderten Absorptionstemperatur ebenfalls nicht zu befriedigen.

Die Aufgabe der vorliegenden Erfindung bestand daher insbesondere darin, ein Verfahren zur kontinuierlichen Abtrennung von Acrylsäure aus dem Reaktionsgasgemisch der katalytischen Gasphasenoxidation von Propylen und/oder Acrolein unter Gegenstromabsorption mit einer hochsiedenden inerten hydrophoben organischen Flüssigkeit zur Verfügung zu stellen, das die Nachteile der Verfahren des Standes der Technik nicht aufweist.

Eine einfache Eliminierung des Desorptionsschrittes scheidet als Problemlösung aus, da ein unmittelbares Einleiten des Flüssigkeitsablaufs einer konventionellen Absorptionskolonne in eine Rektifikationskolonne, insbesondere aufgrund des in einem solchen Flüssigkeitsablauf noch enthaltenen Anteils von bei Normaldruck niedriger als Acrylsäure siedenden, von Acrylsäure nicht sehr verschiedenen (bei 20°C und Normaldruck (1 atm) flüssigen), Nebenkomponenten (Niedrigsieder) das herkömmliche Trennvermögen einer Rektifikationskolonne übersteigen würde (z.B. wären mit der erhöhten Polymerisationsneigung der Acrylsäure unvereinbar hohe Sumpftemperaturen erforderlich, um die Anzahl der benötigten Trennstufen thermisch zu bewältigen).

Als Lösung der gestellten Aufgabe wurde ein Verfahren zur kontinuierlichen Abtrennung von Acrylsäure aus dem Reaktionsgasgemisch der katalytischen Gasphasenoxidation von Propylen und/oder Acrolein durch Gegenstromabsorption mit einer hochsiedenden inerten hydrophoben organischen Flüssigkeit, bei dem man das Reaktionsgasgemisch in einer Absorptionskolonne zu der absteigenden hochsiedenden inerten hydrophoben organischen Flüssigkeit im Gegenstrom führt und anschließend aus dem die Acrylsäure enthaltenden Flüssigkeitsablauf der Absorptionskolonne die Acrylsäure rektifikativ abtrennt, gefunden, das dadurch gekennzeichnet ist, daß man dem in der Absorptionskolonne in natürlicher Weise erfolgenden Absorptionsprozeß einen Rektifikationsprozeß überlagert, indem man längs der Absorptionskolonne einen Teil der absteigenden Flüssigphase entnimmt, diesen Teil abkühlt und anschließend abgekühlt in die Absorptionskolonne so zurückführt, daß die unterste theoretische Trennstufe eine Temperatur von 110 bis 125°C aufweist und die Temperatur auf der obersten theoretischen Trennstufe 5 bis 15°C oberhalb der Aufgabetemperatur des Absorptionsmittels liegt.

Als Absorptionskolonne kommen alle gängigen Typen in Betracht. D.h. die Absorptionskolonne kann z.B. eine Ventilboden-, Glockenboden-, Füllkörper- oder Packungskolonne sein. Packungskolonnen sind bevorzugt, wobei unter diesen Packungskolonnen mit geordneten Packungen besonders vorteilhaft sind. Packungskolonnen mit geordneten Packungen sind dem Fachmann an sich bekannt und z.B. in Chem. -Ing. Tech. 58 (1986) Nr. 1, S. 19-31 sowie in der Technischen Rundschau Sulzer 2/1979, S. 49ff der Gebrüder Sulzer Aktiengesellschaft in CH-Winterthur beschrieben. Erfindungsgemäß hat sich die Anwendung von Blech- und Plattenpackungen als günstig erwiesen. Besonders vorteilhaft ist die Anwendung von Mellapak® Kolonnenpackungen der Firma Sulzer AG, z.B. solche vom Typ 250 Y oder vom Typ 125 Y. Geordnete Packungen zeichnen sich insbesondere durch einen geringen Druckverlust aus und gewährleisten geringe Verweilzeiten, was im Hinblick auf die hohe Polymerisationsneigung der Acrylsäure von Vorteil ist. Zweckmäßigerweise umfaßt die Absorptionskolonne, je nach Gehalt des Rektionsgasgemisches an unter Normaldruck niedriger als Acrylsäure siedenden, bei 20°C und Normaldruck flüssigen, Nebenprodukten (Niedrigsieder), insbesondere Essigsäure und Wasser, 5 bis 20 theoretische Trennstufen. Üblicherweise enthält das der Gasphasenoxidation entnommene Reaktionsgasgemisch, bezogen auf die darin enthaltene Acrylsäure, 1 bis 10 Gew.-% an Niedrigsiedern.

Das Absorptionsmittel wird üblicherweise am Kopf der Absorptionskolonne aufgegeben, während das gasförmige Reaktionsgemisch normalerweise am unteren Ende der Absorptionskolonne eintritt. Erfindungsgemäß erfolgt die Entnahme der absteigenden Flüssigphase, bezogen auf die Länge der Absorptionskolonne, zweckmäßigerweise etwa in der Mitte und die Rückfuhr wird in der Regel wenig oberhalb der Entnahmestelle durchgeführt.

Als hochsiedende inerte hydrophobe organische Absorptionsflüssigkeit kommen alle diejenigen in Betracht, deren Siedetemperatur bei Normaldruck oberhalb der Siedetemperatur der Acrylsäure liegt und die zu wenigstens 70 Gew.-% aus solchen Molekülen bestehen, die keine nach außen wirkende polare Gruppen enthalten und somit beispielsweise nicht in der Lage sind, Wasserstoffbrücken auszubilden.

Somit kommen als Absorptionsflüssigkeit insbesondere alle diejenigen in Betracht, die in der DE-A 21 36 396 und in der DE-A 43 08 087 empfohlen werden. Dies sind im wesentlichen Flüssigkeiten, deren Siedepunkt bei Normaldruck oberhalb von 160°C liegt. Beispielhaft genannt seien Mittelölfraktionen aus der Paraffindestillation, Diphenylether, Diphenyl, oder Mischungen der vorgenannten Flüssigkeiten wie z.B. ein Gemisch aus 70 bis 75 Gew.-% Diphenylether und 25 bis 30 Gew.-% Diphenyl. Besonders günstig ist die Verwendung eines Gemisches bestehend aus einer Mischung aus 70 bis 75 Gew.-% Diphenylether und 25 bis 30 Gew.-% Diphenyl, sowie, bezogen auf diese Mischung, 0,1 bis 25 Gew.-% o-Dimethylphthalat.

In an diese Absorptionsflüssigkeiten angepaßter Weise wird das den Oxidationsreaktor verlassende, in typischer Weise 200 bis 350°C heiße, Reaktionsgasgemisch vor Eintritt in die Absorptionskolonne in zweckmäßiger Weise auf eine Temperatur von 190 bis 230°C, vorzugsweise von 200 bis 210°C gekühlt. Vorzugsweise erfolgt diese Kühlung, z.B. um eine bei direkter Kühlung mit einer Flüssigkeit häufig eintretende Aerosobildung zu vermeiden, durch indirekten Wärmeaustausch.

In der Regel wird dieser indirekte Wärmeaustausch in einem Rohrbündelwärmeaustauscher durchgeführt. Als Kühlmedium kann beispielsweise Siedewasser (unter Drücken von bis zu 12 bar stehend) oder auch eine der möglichen Absorptionsflüssigkeiten verwendet werden. Wesentlich ist, daß im Rahmen dieser Kühlung der Taupunkt des Reaktionsgasgemisches nicht unterschritten wird. Weist das den Oxidationsreaktor verlassende Reaktionsgasgemisch bereits eine geeignete Absorptionstemperatur auf, entfällt der Schritt der indirekten Kühlung selbstredend.

Die Aufgabe des Absorptionsmittels am Kolonnenkopf erfolgt normalerweise in flüssiger, auf den Querschnitt der Packungskolonne verteilter Form (aufregnen), wobei die Absorptionsflüssigkeit in der Regel eine Temperatur von 50 bis 100, vorzugsweise von 50 bis 70°C aufweist.

In der Regel wird die Absorptionskolonne bei geringem Überdruck betrieben. Vorzugsweise liegt der Arbeitsdruck zwischen einem und zwei bar.

Beim erfindungsgemäßen Verfahren befindet sich die Wandung der Absorptionskolonne selbst normalerweise in unmittelbarem Kontakt mit der sie umgebenden Atmosphäre, die üblicherweise Raumtemperatur aufweist. Als Wandungsmaterial kommen prinzipiell alle geeigneten Materialien in Betracht, d.h. z.B. Keramik oder Glas, insbesondere aber Edelstahl. Hinsichtlich der Auswahl des Wandmaterials stehen Eigenschaften wie Korrosionsbeständigkeit und mechanische Beständigkeit im Vordergrund. Typische Edelstahlwanddicken liegen bei 4 bis 20 mm.

Erfindungsgemäß werden Betriebsdruck, Druckverlust der Kolonne und die Art des verwendeten Absorptionsmittels so gewählt, daß die unterste theoretische Trennstufe eine Temperatur von 110 bis 125°C, vorzugsweise von 115 bis 120°C, aufweist und die Temperatur auf der obersten theoretischen Trennstufe 5 bis 15°C, vorzugsweise 10°C oberhalb der Aufgabetemperatur des Absorptionsmittels liegt. Dies kann z.B. dadurch erfolgen, daß nur eine relativ kleine Flüssigkeitsmenge entnommen und stark gekühlt zurückgeführt wird, oder daß man eine relativ große Flüssigkeitsmenge entnimmt und nur mäßig kühlt. Eine solche Entnahme von absteigender Flüssigkeit läßt sich z.B. durch einen überdachten Kamineinbau realisieren, dessen Kamin infolge der Überdachung nur für das aufsteigende Dampf/Gas-Gemisch durchlässig ist, dessen Kaminboden, auf dem die absteigende Flüssigkeit aufläuft, jedoch Überlaufrohre (vorzugsweise vier Überlaufrohre symmetrisch um den in der Mitte befindlichen Kamin angeordnet) aufweist. Die bis auf Überlaufniveau aufgelaufene Flüssigkeit kann dem Kaminboden in an sich bekannter Weise entnommen werden.

Die Sumpfflüssigkeit der Absorptionskolonne enthält bei dem erfindungsgemäßen Verfahren die Niedrigsieder, insbesondere Essigsäure, nur noch in einem außerordentlich effizienten Maß entreichert (bezogen auf die darin enthaltene Acrylsäure in der Regel nur noch 0,1 bis 1 Gew.-%, d.h. etwa ein Zehntel des Ursprungsgehaltes), das es gestattet, die Sumpfflüssigkeit, ohne den Zwischenschritt einer Desorption durchlaufen zu müssen, unmittelbar in an sich bekannter Weise rektifikativ aufzuarbeiten und dabei eine Acrylsäure abzutrennen, deren Reinheit wenigstens 99,7 Gew.-% beträgt.

Vorzugsweise wird das erfindungsgemäße Verfahren kontinuierlich durchgeführt. Dabei beträgt die am Kolonnenkopf der Absorptionskolonne zugegebene hochsiedende hydrophobe inerte organische Flüssigkeitsmenge, bezogen auf die in einem identischen Zeitraum über das aufzuarbeitende Reaktionsgasgemisch zugeführte Gewichtsmenge an Acrylsäure, in der Regel das 4- bis 8-fache.

Die im Rahmen der rektifikativen Abtrennung der Acrylsäure aus der Sumpfflüssigkeit der Absorptionskolonne anfallende hochsiedende organische Flüssigkeit kann bei einer kontinuierlichen Ausführungsweise des erfindungsgemäßen Verfahrens unmittelbar als Absorptionsmittel wieder verwendet und am Kopf der Absorptionskolonne rückgeführt werden. Um die Betriebszeiten der Anlage zu erhöhen ist es dabei gegebenenfalls empfehlenswert, einen Teilstrom dieser hochsiedenden organischen Flüssigkeit abzuzweigen und erst nach Abtrennung von darin angereichert enthaltenen, praktisch nicht verdampfbaren, Verunreinigungen rückzuführen.

Bei dieser Ausführungsvariante des erfindungsgemäßen Verfahrens gibt man den selbstverständlich bei allen Arbeitsschritten (ausgenommen die Kühlung des Reaktionsgasgemisches auf Absorptionstemperatur) benötigten Polymerisationsinhibitor, z.B. Phenothiazin, zweckmäßigerweise am Kopf der die Acrylsäure abtrennenden Rektifikationskolonne dem Gesamtsystem zu.

Im übrigen wird empfohlen, zur rektifikativen Abtrennung der Acrylsäure aus der Sumpfflüssigkeit der Absorptionskolonne ebenfalls eine Kolonne mit geordneten Packungen anzuwenden und dabei Acrylsäure über seitlichen Abzug zu entnehmen.

Besonders vorteilhaft wird das erfindungsgemäße Verfahren auf solche Reaktionsgasgemische der gasphasenkatalytisch oxidativen Herstellung von Acrylsäure angewendet, wie sie z.B. gemäß der DE-A 42 20 859 erhalten werden.

Im Prinzip kann das im Rahmen des erfindungsgemäßen Verfahrens am Kopf der Absorptionskolonne austretende Dampf/Gas-Gemisch abgeführt werden.

In zweckmäßiger Weise wird man jedoch der vorstehend beschriebenen Hauptkolonne eine Nebenkolonne (es kommen prinzipiell dieselben Kolonnentypen wie für die Hauptkolonne in Betracht) aufsetzen, wobei der Übergang von der einen Kolonne in die andere nur für das aufsteigende Dampf/Gas-Gemisch ermöglicht wird, in welcher man das aufsteigende Dampf/Gas-Gemisch zu am Kopf dieser Nebenkolonne aufgegebenem Kaltwasser im Gegenstrom führt. Der Hauptzweck der Nebenkolonne besteht darin, die bei der Temperatur des aufgegebenen Kaltwassers kondensierbaren Bestandteile des in die Nebenkolonne aufsteigenden Dampf/Gas-Gemisches zu kondensieren, so daß am Kopf dieser Kolonne ein Gemisch abgeführt führt, das im wesentlichen nur noch die im Rahmen der gasphasenkatalytisch oxidativen Herstellung der Acrylsäure mitverwendeten inerten Verdünnungsgase wie N₂ etc. sowie im Rahmen der Gasphasenoxidation durch z.B. Vollverbrennung zu Kohlenoxiden entstandene Reaktionsgasgemischanteile enthält.

Im Prinzip ist es daher ausreichend, wenn die Nebenkolonne eine theoretische Kondensationsstufe umfaßt. Vorzugsweise wird die Nebenkolonne jedoch so ausgelegt, daß sie 2 bis 4 theoretische Kondensationsstufen umfaßt. Die im Sumpf der Nebenkolonne auflaufende absteigende Flüssigkeit trennt sich in der Regel in eine überwiegend wäßrige Phase, die insbesondere erhebliche Mengen der meist gut in Wasser löslichen niedriger als Acrylsäure siedenden Nebenprodukte wie Essigsäure enthält und in eine hauptsächlich Absorptionsmittel umfassende, in der Regel spezifisch schwerere, organische Phase. Bei kontinuierlicher Betriebsweise wird man zweckmäßig einen Teil der wäßrigen Phase, und mit diesem die darin enthaltenen Niedrigsieder, abtrennen und abführen, während die verbleibende Restmenge gekühlt und am Kopf der Nebenkolonne anstelle des bei Verfahrensbeginn aufgegebenen Frischwassers in die Nebenkolonne im Kreis zurückgeführt wird. Entsprechend wird normalerweise auf die erforderliche Kaltwassertemperatur gekühlt, d.h. in der Regel auf 5 bis 30°C, vorzugsweise auf 5 bis 25°C und die nach Abtrennung abgeführte Menge an wäßriger Phase so bemessen, daß die darin enthaltene Menge an Wasser derjenigen aus dem aus der Hauptkolonne aufsteigenden Dampf/Gas-Gemisch in der Nebenkolonne auskondensierten Menge Wasserdampf entspricht. Die organische Phase aus dem Sumpf der Nebenkolonne wird normalerweise in die Hauptkolonne, vorzugsweise über Kopf, rückgeführt.

In der Nebenkolonne überlagern sich Kondensations-, Absorptions- und Phasenzerfallsprozesse. Vorzugsweise wird man als Nebenkolonne ebenfalls eine Packungskolonne mit geordneten Packungen anwenden.

Die am Kopf der Nebenkolonne aufgegebene Kaltwassermenge wird so bemessen, daß sie einerseits den gewünschten Kondensationseffekt und andererseits kein Fluten der Kolonne bewirkt. Diese Menge hängt somit in natürlicher Weise von der gewählten Aufgabetemperatur ab.

In der Praxis wird man das übereinander von Haupt- und Nebenkolonne zweckmäßig in Gestalt einer einzigen Kolonne ausführen, in welcher ein unterer Abschnitt (entspricht der Hauptkolonne) von einem oberen Abschnitt (entspricht der Nebenkolonne) in lediglich für das aufsteigende Dampf/Gas-Gemisch durchlässiger Weise abgetrennt ist. Dies läßt sich z.B. durch einen Kamineinbau mit überdachtem Kamin und undurchlässigem Kaminboden realisieren. Der Kaminboden dient somit als Sammelboden für die absteigende Flüssigphase des oberen Kolonnenabschnitts, welche sich auf diesem Sammelboden, wie bereits beschrieben, in eine überwiegend organische und eine überwiegend wäßrige Phase scheidet.

Als eine besonders bevorzugte Lösung der vorliegender Erfindung zugrunde liegenden Aufgabe wird daher ein Verfahren zur Abtrennung von Acrylsäure aus dem Reaktionsgasgemisch der katalytischen Gasphasenoxidation von Propen und/oder Acrolein durch Gegenstromabsorption mit einer hochsiedenden inerten hydrophoben organischen Flüssigkeit, bei dem man das Reaktionsgasgemisch in einer Absorptionskolonne zu der absteigenden hochsiedenden inerten hydrophoben organischen Flüssigkeit im Gegenstrom führt und anschließend aus dem die Acrylsäure enthaltenden Flüssigkeitsablauf der Absorptionskolonne die Acrylsäure rektifikativ abtrennt, zur Verfügung gestellt, das dadurch gekennzeichnet ist, daß man das die erforderliche Temperatur aufweisende Reaktionsgasgemisch unterhalb der untersten Packung in eine im wesentlichen durchgehend mit geordneten Packungen ausgestattete Packungskolonne, die sich in zwei Abschnitte gliedert, die voneinander durch eine nur für das aufsteigende Dampf/Gas-Gemisch durchlässige Verbindung getrennt sind, leitet, wobei man im unteren Abschnitt das unterhalb der untersten Packung (Zufuhrstelle 1) zugeführte aufsteigende Reaktionsgasgemisch zu oberhalb der obersten Packung des unteren Abschnitts (Zufuhrstelle 2) auf den Kolonnenquerschnitt verteilt aufgegebenem hochsiedendem hydrophobem organischem Absorptionsmittel im Gegenstrom führt und dabei längs dieses Kolonnenabschnitts einen Teil der absteigenden Flüssigphase entnimmt, abkühlt und im sich von dieser Entnahmestelle (Entnahmestelle 1) bis zur Zufuhrstelle 2 erstreckenden Bereich abgekühlt in die Packungskolonne zurückführt, im oberen Abschnitt der Packungskolonne das durch die entsprechend durchlässige Verbindung aufsteigende Dampf/Gas-Gemisch zu oberhalb der obersten Packung (Zufuhrstelle 3) auf den Kolonnenquerschnitt verteilt aufgegebenem Kaltwasser im Gegenstrom führt, die Verbindung als Sammelstelle für die absteigende Flüssigphase des oberen Kolonnenabschnitts ausgestaltet, welche sich an dieser Sammelstelle in eine überwiegend organische und eine überwiegend wäßrige Phase scheidet, die in der Sammelstelle auflaufende organische Phase dem Sammelboden entnimmt und in den unteren Kolonnenabschnitt zurückführt, die in der Sammelstelle auflaufende wäßrige Phase selbiger ebenfalls entnimmt (Entnahmestelle 2), von der entnommenen wäßrigen Phase einen solchen Teil abtrennt und abführt, daß die in diesem Teil enthaltene Wassermenge der aus dem durch die Trennverbindung aufsteigenden Dampf/Gas-Gemisch auskondensierten Menge Wasserdampf entspricht, die dabei verbleibende Restmenge der entnommenen Wasserphase kühlt und an der Zufuhrstelle 3 in den oberen Kolonnenabschnitt im Kreis zurückführt, am Kolonnenkopf die durch das Kaltwasser nicht verflüssigte Gasphase abführt und den in der Hauptsache aus Acrylsäure und dem hochsiedenden hydrophoben organischen Absorptionsmittel bestehenden Kolonnensumpf direkt in eine Rektifikationskolonne überführt und in selbiger die Acrylsäure aus dieser Sumpfflüssigkeit in an sich bekannter Weise rektifikativ abtrennt.

Vorzugsweise weist vorgenannte Rektifikationskolonne mehr als 20 theoretische Trennstufen auf und wird unter reduziertem Druck betrieben. Besonders zweckmäßig ist es bei einem Kopfdruck unterhalb von 100 mbar zu arbeiten. Besonders günstig ist es zur im Rahmen der Rektifikation erforderlichen Verdampfung Fallfilmverdampfer anzuwenden und so den Verdampfungsprozeß schonend durchzuführen.

Selbstverständlich läßt sich das erfindungsgemäße Verfahren auch zur Abtrennung von Methacrylsäure aus dem Reaktionsgasgemisch der katalytischen Gasphasenoxidation von C₄- oder verkappten C₄-Ausgangsverbindungen (z.B. Buten, tert.-Butenol, Ether von tert.-Butanol, Methacrolein etc., vgl. z.B. DE-A 44 05 059) in entsprechender vorteilhafter Weise anwenden. Die Hauptvorteile des erfindungsgemäßen Verfahrens liegen im minimalen Gesamtenergieeinsatz und der besonders schonenden Behandlung der sehr reaktionsfähigen ungesättigten Carbonsäuren begründet, durch die unkontrollierte Reaktionen gemindert werden.

### Beispiel

Bei einem Verfahren der gasphasenkatalytisch oxidativen Herstellung von Acrylsäure aus Propylen, bei dem als Verdünnungsmittel N₂ verwendet wurde, wurde ein eine Temperatur von 277°C aufweisender Reaktionsgasgemisch erhalten, der nachfolgende Zusammensetzung aufwies:
- 13,5 Gew.-%: Acrylsäure,
- 0,25 Gew.-%: bei Normaldruck höher als Acrylsäure siedende Nebenprodukte (Hochsieder),
- 5,35 Gew.-%: bei Normaldruck niedriger als Acrylsäure siedende Nebenprodukte (Niedrigsieder, z.B. H₂O, Acrolein, Formaldehyd, Acetaldehyd und insbesondere Essigsäure),
- 80,9 Gew.-%: Restgas (im wesentlichen N₂, Spuren von Kohlenoxiden und Propylen).
220,2 kg/h dieses Reaktionsgasgemisches wurde in einem Rohrbündelwärmeaustauscher indirekt auf eine Temperatur von 204°C gekühlt. Als Kühlmedium wurde ein Gemisch bestehend aus 75 Gew.-% Diphenylether und 25 Gew.-% Diphenyl verwendet.

Anschließend wurde das gekühlte Reaktionsgasgemisch in eine mit geordneten Mellapak Packungen der Fa. Sulzer AG, CH-Winterthur ausgestattete, mit Edelstahl bewandete (Wanddicke: 4 mm), Packungskolonne (Innendurchmesser: 316 mm) geleitet, die sich in unmittelbarem Kontakt zur umgebenden Atmosphäre (25°C) befand und wie folgt aufgebaut war:
Auf einem oberhalb der Zufuhrstelle des gekühlten Reaktionsgasgemisches befindlichen Tragrost wurden zunächst 2 der vorgenannten Packungen vom Typ 125 Y und anschließend 16 der vorgenannten Packungen vom Typ 250 Y (der Durchmesser der Einzelpackung betrug 314 mm und die Höhe betrug 210 mm) als ein erster Packungsstapel unmittelbar aufeinandergestapelt. Unterhalb des Tragrostes befand sich zur Aufnahme der Sumpfflüssigkeit ein konstruktiver Anteil einer Länge von 800 mm. Die Zufuhrstelle des gekühlten Reaktionsgemisches (Zufuhrstelle 1) war in der Mitte des konstruktiven Teils angebracht. 50 mm oberhalb des Endes des ersten Packungsstapels war ein über den Kolonnenquerschnitt gehender Sammelboden für die absteigende Flüssigkeit installiert, der in der Mitte zum Durchlaß für das aufsteigende Dampf/Gas-Gemisch einen überdachten Kamin aufwies (Höhe: 400 mm; Durchmesser: 106 mm), um welchen symmetrisch vier Überlaufrohre (Höhe: 300 mm; Durchmesser: 43 mm) angebracht waren.

Von diesem Sammelboden (Entnahmestelle 1) wurden kontinuierlich 1700 kg/h der auf diesem Sammelboden auflaufenden flüssigen Phase mit einer Temperatur von 59,9°C entnommen, auf 42°C gekühlt und unmittelbar über dem Ende des zweiten Packungsstapels mit dieser Temperatur in die Packungskolonne zurückgeführt.

Der zweite Packungsstapel bestand aus drei unmittelbar aufeinandergestapelten geordneten Packungen derselben Sorte (Typ 250 Y), die auf einem oberhalb des zum Sammelboden gehörenden Durchlaßkamins befindlichen Tragerost aufgesetzt waren und trennte die Entnahmestelle 1 von der Rückführstelle. Oberhalb der Rückführstelle waren 13 weitere Packungen des Typs 250 C übereinandergestapelt (dritter Packungsstapel) angebracht. Am Ende dieses Dritten Packungsstapels befand sich die Zufuhrstelle für das Absorptionsmittel (Zuführstelle 2). Als Absorptionsmittel wurde ein Gemisch aus 57,4 Gew.-% Diphenylether, 20,7 Gew.-% Diphenyl und 20 Gew.-% o-Dimethylphthalat verwendet. Es wurde an der Zuführstelle 2 mit einer Temperatur von 52°C und in einer Menge von 160,8 kg/h aufgegeben.

Unmittelbar oberhalb der Zuführstelle 2 befand sich ein Trennkamineinbau, oberhalb dessen weitere 5 aufeinandergestapelte geordnete Packungen derselben Sorte (Typ 250 Y) angebracht waren (vierter Packungsstapel). Unmittelbar über dem Ende des vierten Packungsstapels befand sich die Zufuhrstelle des Kaltwasserkreislaufes (Zuführstelle 3). Vom Sammelboden des Trennkamineinbaus wurden 370 kg/h wäßrige Phase entnommen (Entnahmestelle 2) und davon 6,7 kg/h abgetrennt und abgeführt. Die Restmenge wurde an der Zuführstelle 3 rückgeführt. Die Entnahmetemperatur betrug 40,5°C, vor Rückführung wurde auf 25°C gekühlt. Die entnommene wäßrige Phase enthielt 6,9 Gew.-% Acrylsäure (Verlust) und 93,1 Gew.-% an Wasser mit Niedrigsiedern. Die Packungskolonne war oben offen. Über diese Öffnung wurden 181,8 g gasförmiger Verbindungen mit einer Temperatur von 26,6°C abgegeben. Dieses Abgas bestand aus 2 Gew.-% Wasserdampf, > 97,8 Gew.-% Restgas, < 0,1 Gew.-% Acrylsäure und < 0,1 Gew.-% Absorptionsmittel. Der Druck am Kolonnenkopf betrug 1,15 bar, an der Zufuhrstelle 1 1,24 bar. Die an der Entnahmestelle 2 auflaufende organische Phase wurde an der Zufuhrstelle 2 in die Verstärkungssäule rückgeführt.

Die Entnahme der Sumpfflüssigkeit betrug 192,5 kg/h, die zugehörige Entnahmetemperatur betrug 118,4°C. Die Zusammensetzung der Sumpfflüssigkeit war: 15,2 Gew.-% Acrylsäure, 84,35 Gew.-% Absorptionsmittel, 0,29 Gew.-% höher als Acrylsäure siedende Bestandteile (Maleinsäureanhydrid, Phthalsäureanhydrid etc.) und 0,16 Gew.-% Niedrigsieder inklusive H₂O.

Die entnommene Sumpfflüssigkeit wurde mit geordneten Packungen in eine konventionelle Rektifikationskolonne (Packungskolonne mit geordneten Packungen) geführt und in an sich bekannter Weise rektifikativ unter reduziertem Druck aufgetrennt. Acrylsäure wurde dabei über Seitenabzug in einer Reinheit > 99,7 Gew.-% gewonnen. Am Kolonnenkopf fielen geringe Mengen Niedrigsieder an und die Sumpfflüssigkeit wurde als Absorptionsmittel wiederverwendet. In entsprechender Weise wurde das zur Polymerisationsinhibierung verwendete Phenothiazin am Kolonnenkopf dieser Rektifikationskolonne aufgegeben.

## Patentansprüche

1. Verfahren zur Abtrennung von (Meth)acrylsäure aus dem Reaktionsgasgemisch der katalytischen Gasphasenoxidation durch Gegenstromabsorption mit einer hochsiedenden inerten hydrophoben organischen Flüssigkeit, bei dem man das Reaktionsgasgemisch in einer Absorptionskolonne zu der absteigenden hochsiedenden inerten hydrophoben organischen Flüssigkeit im Gegenstrom führt und anschließend aus dem die (Meth)acrylsäure enthaltenden Flüssigkeitsablauf der Absorptionskolonne die (Meth)acrylsäure rektifikativ abtrennt, dadurch gekennzeichnet, daß man dem Absorptionsprozeß einen Rektifikationsprozeß überlagert, in dem man längs der Absorptionskolonne einen Teil der absteigenden Flüssigphase entnimmt, diesen Teil abkühlt und anschließend abgekühlt in die Absorptionskolonne so zurückführt, daß die unterste theoretische Trennstufe eine Temperatur von 110 bis 125°C aufweist und die Temperatur auf der obersten theoretischen Trennstufe 5 bis 15°C oberhalb der Aufgabetemperatur des Absorptionsmittels liegt.

## Claims

1. A process for separating (meth)acrylic acid from the reaction gas mixture from catalytic gas-phase oxidation by countercurrent absorption using a high-boiling inert hydrophobic organic liquid, in which process the reaction gas mixture is passed through an absorption column in countercurrent to the descending high-boiling inert hydrophobic organic liquid and the (meth)acrylic acid is subsequently separated by rectification from the (meth)acrylic acid-containing liquid discharge from the absorption column, wherein a rectification process is superimposed on the absorption process, by taking off a part of the descending liquid phase along the absorption column, cooling this part and subsequently returning the cooled liquid to the absorption column in such a way that the lowest theoretical plate is at from 110 to 125°C and the uppermost theoretical plate is at a temperature which is from 5 to 15°C above the temperature at which the absorption medium is introduced.

## Revendications

1. Procédé pour la séparation d'acide (méth)acrylique à partir du mélange réactionnel gazeux de l'oxydation catalytique en phase gazeuse, par absorption à contre-courant avec un liquide organique hydrophobe inerte à haut point d'ébullition, dans lequel on fait passer le mélange réactionnel gazeux dans une colonne d'absorption, à contre-courant par rapport au liquide organique hydrophobe à haut point d'ébullition, descendant, et ensuite on sépare par rectification l'acide (méth)acrylique de l'effluent liquide de la colonne d'absorption, contenant l'acide (méth)acrylique, caractérisé en ce que l'on superpose au processus d'absorption un processus de rectification dans lequel on prélève le long de la colonne d'absorption une partie de la phase liquide descendante, on refroidit cette partie et ensuite on la recycle, refroidie, dans la colonne d'absorption, de manière que l'étage de séparation théorique le plus bas présente une température de 110 à 125°C et que la température de l'étage de séparation théorique le plus élevé soit supérieure de 5 à 15°C à la température d'arrivée de l'agent d'absorption.
